# EUROPÄISCHE PATENTSCHRIFT

(11) **EP 0 451 714 B1**
(45) Veröffentlichungstag und Bekanntmachung des Hinweises auf die Patenterteilung: **05.01.1994**
(21) Anmeldenummer: 91105384.1
(22) Anmeldetag: 05.04.1991
(51) Int. Cl.: C07C 65/34, C07C 51/373

(54) **Verfahren zur Acylierung von Alkylaromaten**
Process for the acylation of alkyl aromatics
Procédé pour l'acylation de composés aromatiques alkylés

(30) Priorität: 12.04.1990 DE 4011916
(43) Veröffentlichungstag der Anmeldung: 16.10.1991
(73) Patentinhaber: BASF Aktiengesellschaft, 67063 Ludwigshafen (DE)
(72) Erfinder: Henkelmann, Jochem, Dr., W-6700 Ludwigshafen (DE); Siegel, Hardo, Dr., W-6720 Speyer (DE)

(56) Entgegenhaltungen:
- EP-A- 0 248 423

## Beschreibung

Die vorliegende Erfindung betrifft ein verbessertes Verfahren zur Acylierung von Alkylaromaten mit Carbonsäurederivaten nach Friedel-Crafts in Gegenwart von Friedel-Crafts-Katalysatoren und aluminiumorganischen Verbindungen.

Die Friedel-Crafts-Acylierung von Aromaten mit Carbonsäurederivaten ist allgemein bekannt, jedoch hat man die Probleme bei der Acylierung von Alkylaromaten nicht gänzlich befriedigend lösen können. Bei der Verwendung der üblichen Friedel-Crafts-Katalysatoren wie den Aluminiumhalogeniden wird Halogenwasserstoff freigesetzt, welcher die Bildung harzartiger Nebenprodukte sowie die Abspaltung und Isomerisierung der Alkylgruppen begünstigt (C.A. Olah, Friedel-Crafts and Related Reactions, Bd. 1, Seite 207 und Bd. 3, Teil I, Seite 550 ff., Interscience 1964). Als vorteilhaft bei der Beseitigung des Halogenwasserstoffs hat sich die Mitverwendung von Metallalkylen bzw. Metallalkylhalogeniden erwiesen (EP-A-0248423). Diese Verbindungen katalysieren jedoch selbst die Isomerisierung von Alkylaromaten.

Der Erfindung lag daher die Aufgabe zugrunde, diese Nebenreaktionen noch wirksamer als bisher zurückzudrängen.

Demgemäß wurde ein Verfahren zur Acylierung von Alkylaromaten mit Carbonsäurederivaten nach Friedel-Crafts in Gegenwart von Friedel-Crafts-Katalysatoren und aluminiumorganischen Verbindungen gefunden, welches dadurch gekennzeichnet ist, daß man als aluminiumorganische Verbindungen Aluminoxane verwendet.

Als Aluminoxane eignen sich insbesondere Verbindungen der allgemeinen Formel I 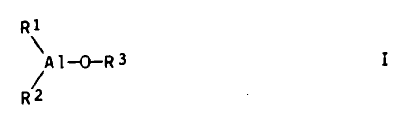
in der
- R¹: für eine Alkylgruppe mit 1 bis 12, vorzugsweise 1 bis 6, insbesondere 1 bis 4 C-Atomen wie n-Propyl, iso-Propyl, iso-Butyl und besonders bevorzugt Methyl, Ethyl oder n-Butyl steht,
- R²: für eine Alkyl- oder Alkoxygruppe mit 1 bis 12, vorzugsweise 1 bis 6, insbesondere 1 bis 4 Kohlenstoffatomen, wie einen der Reste R¹ oder die Propoxy- und insbesondere Methoxy-, Ethoxy- oder Butoxygruppe und
- R³: für eine Alkylgruppe mit der Bedeutung wie R¹ oder einen Rest Ia

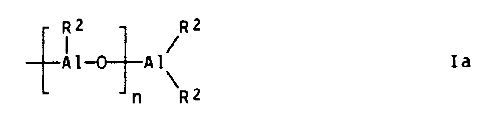

in dem n für einen Mittelwert von 0 bis 50, insbesondere 15 bis 40, steht. Bei den durch den Rest Ia gekennzeichneten Aluminoxanen handelt es sich definitionsgemäß um lineare Verbindungen. Solche Aluminoxane sind beispielsweise nach den Verfahren von Houben-Weyl, Methoden der Organischen Chemie, Bd. XIII/4, Seiten 78-80, Thieme, Stuttgart, 1970, und US-A 4 952 714 erhältlich.

Weiterhin kommen aber auch cyclische Verbindungen Ib des ähnlichen Aufbaus 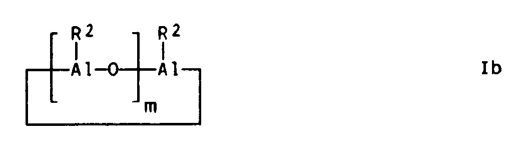
in Betracht, wobei m einen Mittelwert von 3 bis 12 hat. Die linearen und cyclischen Aluminoxane können durch vorsichtige Hydrolyse von Aluminiumalkylverbindungen erhalten werden (Houben-Weyl, Methoden der Org. Chemie, Bd. XIII/4, 1970, Seiten 76-78).

Die Wirkung der Aluminoxane beruht darauf, daß der vor der Friedel-Crafts-Reaktion vorhandene oder während der Reaktion gebildete Halogenwasserstoff HX mit den Alkyl-Al-Gruppen unter Bildung von Kohlenwasserstoffen und Aluminiumhalogeniden reagiert 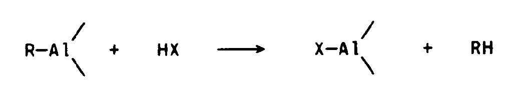
und somit abgefangen wird.

Demnach entspricht die theoretisch erforderliche Menge an I der molaren Menge an Alkyl-Aluminiumbindungen, die ihrerseits äquimolar zum abgespaltenen Halogenwasserstoff ist. Da aber ein Teil des Halogenwasserstoffs sofort aus dem Reaktionsgemisch entweicht, genügen häufig geringere Mengen. In der Regel beträgt die Menge an I 0,1 bis 1,1 mol Alkyl-Aluminiumbindungen pro Mol des Carbonsäurederivats.

Da die entstehende Verbindungen 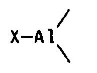
ihrerseits als Friedel-Crafts-Katalysatoren wirken, ermäßigt sich der Bedarf an dem primär eingesetzten Katalysator, z.B. AlCl₃, entsprechend. Im allgemeinen benötigt man nur 60 bis 95 % des primär eingesetzten Katalysators, verglichen mit der Menge, die ohne die Mitverwendung von I für die Acylierung erforderlich wäre.

Größere Mengen an Friedel-Crafts-Katalystoren sind ebenfalls möglich.

Als Friedel-Crafts-Katalysatoren eignen sich die üblicherweise in diesem Verfahren verwendeten Verbindungen wie FeCl₃, ZnCl₂ oder TiCl₄, vorzugsweise die Aluminiumhalogenide, insbesondere Aluminiumbromid und vor allem Aluminiumchlorid.

üblicherweise wird der Friedel-Crafts-Katalysator für eine vollständige Umsetzung in Mengen von 1 bis 1,5 mol, vorzugsweise 1,1 bis 1,2 mol pro Äquivalent Carbonsäurehalogenid bzw. 2 bis 2,5 mol, vorzugsweise 2,1 bis 2,2 mol pro Äquivalent Carbonsäureanhydrid eingesetzt. Größere Mengen sind möglich, bringen aber in der Regel keine weiteren Vorteile mehr.

Die Reaktion kann ohne oder bevorzugt in Gegenwart eines Lösungsmittels durchgeführt werden. Dabei kommen die üblicherweise bei Friedel-Crafts-Reaktionen verwendeten wasserunlöslichen aprotischen Lösungsmittel wie Chlorbenzol, Dichlorbenzol, 1,2-Dichlorethan, Schwefelkohlenstoff, Nitromethan und Nitrobenzol in Betracht. Die Lösungsmittelmenge ist nicht kritisch, beträgt aber im allgemeinen 100 bis 1000 g pro Mol des Alkylaromaten. Größere Mengen sind möglich, bringen aber im allgemeinen keine Vorteile mehr.

Die Acylierung findet in an sich bekannter Weise statt, wobei die Ausgangsstoffe bei Temperaturen von -20 bis 100°C, vorzugsweise 0 bis 60°C, insbesondere 10 bis 40°C bei vermindertem, erhöhtem oder vorzugsweise bei Normaldruck umgesetzt werden. Dabei wird das Acylierungsmittel normalerweise in dem Lösungsmittel vorgelegt, wonach der Friedel-Crafts-Katalysator und der Alkylaromat mit dem Aluminoxan zugegeben werden.

Die Aufarbeitung bietet methodisch keine Besonderheiten und erfolgt in an sich bekannter Weise, indem man das Reaktionsgemisch auf Wasser oder Eis gießt und das acylierte Produkt aus der organischen Phase isoliert.

Als Ausgangsverbindungen kommen prinzipiell alle alkylierten isocyclischen und heterocyclischen Aromaten wie alkylsubstituierte Benzole, Naphthaline, Anthracene, Furane, Benzofurane und Thiophene in Betracht. Alkylreste sind z.B. solche mit 1 bis 20, insbesondere 1 bis 12, vorzugsweise 1 bis 8 Kohlenstoffatomen. Der Alkylrest wie auch der aromatische Kern können weitere Substituenten wie C₁- bis C₄-Alkyl- oder Alkoxygruppen, Halogen wie Chlor oder Brom oder Hydroxygruppen enthalten. Die aliphatischen Reste können auch Doppel- oder Dreifachbindungen enthalten. Besonders bevorzugt werden Alkylbenzole mit 1 oder 2 verzweigten Alkylresten umgesetzt.

Beispielsweise seien folgende Verbindungen genannt:

Toluol, Ethylbenzol, n-Propylbenzol, n-Butylbenzol, n-Pentylbenzol, n-Hexylbenzol und n-Octylbenzol, insbesondere iso-Propylbenzol, sek.-Butylbenzol, tert.-Butylbenzol, (2-Methylbutyl)-benzol, (3-Methyl-butyl)-benzol, (1-Methylbutyl)-benzol, (1,1-DImethylpropyl)-benzol, (1,1-Dimethylbutyl)-benzol, (1-Methylpentyl)-benzol, (1-Ethyl-1-methyl-butyl)-benzol, (1-Ethylhexyl)-benzol, 1,2-Dimethylbenzol und 1,4-Diethyl-benzol sowie vorzugsweise tert.-Amylbenzol.

Hinsichtlich der Acylierungsmittel unterliegt das erfindungsgemäße Verfahren nach den bisherigen Beobachtungen keinen Beschränkungen. So eignen sich die Fluoride, insbesondere die Bromide und Chloride und die Anhydride aliphatischer, cycloaliphatischer und insbesondere aromatische Carbonsäuren. So kommen Derivate aromatischer Carbonsäuren wie Benzoylchlorid, Benzoesäureanhydrid und besonders bevorzugt Phthalsäureanhydrid, alkylierte aromatische Carbonsäurederivate wie p-Methylbenzoylchlorid und p-tert.-Butylbenzoylchlorid, alkoxylierte Carbonsäurederivate wie 3-Methoxybenzoylchlorid oder 3-Phenoxybenzoylchlorid, halogenierte aromatische Carbonsäurederivate wie o-Chlorbenzoylchlorid und 2,6-Dichlorbenzoylchlorid und p-Nitrobenzoylchlorid, araliphatische Carbonsäurederivate wie Phenylessigsäurechlorid und 4-Chloressigsäurechlorid und heterocyclische Carbonsäurederivate wie Furan-2-carbonsäurechlorid und Thiophen-2-carbon-säurechlorid in Betracht.

Besondere Bedeutung hat das erfindungsgemäße Verfahren für die Herstellung von 4-(2'-Carboxybenzyl)-tert.-amylbenzol aus tert.-Amylbenzol und Phthalsäureanhydrid, das zu dem wichtigen 2-tert. -Amylanthrachinon weiter umgesetzt wird, welches seinerseits als Sauerstoffüberträger für die Herstellung von Wasserstoffperoxid nach dem Riedl-Pfleiderer-Verfahren dient.

### Beispiel

Eine Lösung aus 74 g (0,5 mol) Phthalsäureanhydrid und 200 ml o-Dichlorbenzol wurde zunächst bei 15 bis 20°C in kleinen Portionen mit 128 g (1,05 mol) Aluminiumtrichlorid und danach im Laufe von 5 Stunden mit einer Lösung aus 74 g (0,5 mol) tert.-Amylbenzol und 27 g des Methylaluminoxans 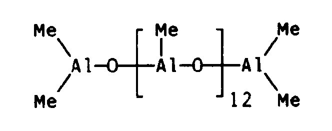
(entsprechend 0,5 mol Alkylaluminiumbindungen) versetzt und anschließend noch 2 Stunden bei Raumtemperatur gerührt.

Die übliche Aufarbeitung der Reaktionsansätze lieferte das 4-(2'-Carboxybenzoyl)-tert.-amylbenzol in einer Ausbeute von 90 %.

Das gleiche Ergebnis wurde bei Verwendung des entsprechenden Ethylaluminoxans in gleicher molarer Menge erzielt.

## Patentansprüche

1. Verfahren zur Acylierung von Alkylaromaten mit Carbonsäurederivaten nach Friedel-Crafts in Gegenwart von Friedel-Crafts-Katalysatoren und aluminiumorganischen Verbindungen, dadurch gekennzeichnet, daß man als aluminiumorganische Verbindungen Aluminoxane verwendet.

2. Verfahren nach Anspruch 1, dadurch gekennzeichnet, daß man als Aluminoxane Verbindungen der allgemeinen Formel I 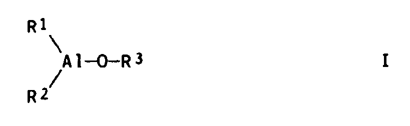 verwendet, in der die Substituenten folgende Bedeutung haben:
R¹ eine C₁-C₁₂-Alkylgruppe
R² eine C₁-C₁₂-Alkyl- oder Alkoxygruppe und
R³ R¹ oder einen Rest Ia 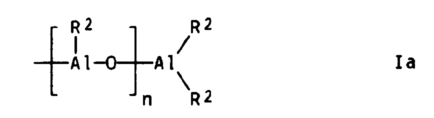
wobei n für 0 bis 50 steht.

3. Verfahren nach Anspruch 1 oder 2, dadurch gekennzeichnet, daß man pro Mol des Carbonsäurederivates 0,1 bis 1,1 mol Alkyl-Aluminiumbindungen einsetzt.

4. Verfahren nach den Ansprüchen 1 bis 3, dadurch gekennzeichnet, daß man als Friedel-Crafts-Katalysatoren Aluminiumchlorid oder -bromid verwendet.

5. Verfahren nach den Ansprüchen 1 bis 4, dadurch gekennzeichnet, daß man es auf die Acylierung von tert.-Amylbenzol mit Phthalsäureanhydrid anwendet.

## Claims

1. A process for Friedel-Crafts acylation of alkylaromatics with carboxylic acid derivatives in the presence of Friedel-Crafts catalysts and organoaluminum compounds, which comprises using aluminoxanes as organoaluminum compounds.

2. A process as claimed in claim 1, wherein the aluminoxane used are compounds of the general formula I 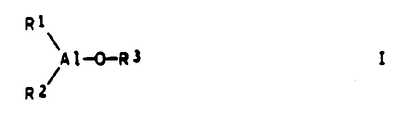 where the substituents have the following meanings:
R¹ is C₁-C₁₂-alkyl,
R₂ is C₁-C₁₂-alkyl or alkoxy and
R³ is R¹ or a radical Ia 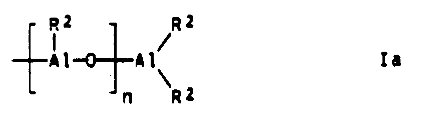 where n is 0 to 50.

3. A process as claimed in claim 1 or 2, wherein from 0.1 to 1.1 mol of alkylaluminum compounds are employed per mole of the carboxylic acid derivative.

4. A process as claimed in any of claims 1 to 3, wherein aluminum chloride or bromide is used as the Friedel-Crafts catalyst.

5. A process as claimed in any of claims 1 to 4, which is used in the acylation of tert-amylbenzene with phthalic anhydride.

## Revendications

1. Procédé pour acyler des composés alkylaromatiques avec des dérivés d'acides carboxyliques par la réaction de Friedel-Crafts en présence de catalyseurs de Friedel-Crafts et de composés organoaluminiques caractérisé en ce qu'on utilise, comme composés organoaluminiques, des aluminoxanes.

2. Procédé selon la revendication 1, caractérisé en ce qu'on utilise comme aluminoxanes des composés répondant à la formule générale I 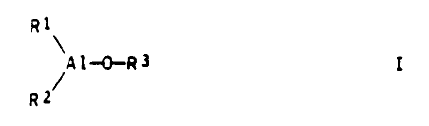 dans laquelle les substituants ont les significations suivantes :
R¹ est un groupe alkyle en C₁-C₁₂ ,
R² est un groupe alkyle ou alcoxy en C₁-C₁₂, et
R³ est égal à R¹ ou est un radical Ia 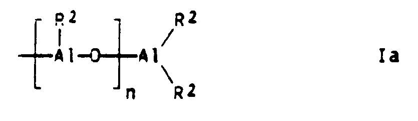 où n vaut de 0 à 50.

3. Procédé selon la revendication 1 ou 2, caractérisé en ce qu'on utilise, par mole du dérivé d'acide carboxylique, de 0,1 à 1,1 mole de liaisons alkyle-aluminium.

4. Procédé selon les revendications 1 à 3, caractérisé en ce qu'on utilise, comme catalyseurs de Friedel-Crafts, du chlorure ou du bromure d'aluminium.

5. Procédé selon les revendications 1 à 4, caractérisé en ce qu'on l'utilise pour acyler du tert-amylbenzène avec de l'anhydride phtalique.
